# EUROPEAN PATENT SPECIFICATION

(11) **EP 1 308 507 B1**
(45) Date of publication and mention of the grant of the patent: **02.01.2008**
(21) Application number: 02257622.7
(22) Date of filing: 04.11.2002
(51) Int. Cl.: C12N 5/20, C07K 16/10, C07K 14/18, G01N 33/576, G01N 33/577

(54) **HCV anti-core monoclonal antibody**
Monoklonales Antikörper gegen das HCV Kernantigen
Anticorps monoclonal contre des antigènes du noyau de CHV

(30) Priority: 05.11.2001 US 337453 P; 10.10.2002 US 268561
(43) Date of publication of application: 07.05.2003
(60) Divisional application: 07075928.7
(73) Proprietor: Ortho Clinical Diagnostics Inc., Rochester, New York 14626 (US)
(72) Inventor: Bahl, Chander, Flemington, NJ 08822 (US)
(74) Representative: Mercer, Christopher Paul

(56) References cited:
- EP-A- 0 388 232
- EP-A- 0 537 856
- WO-A-00/63444
- FR-A- 2 775 690

## Description

### Background of the Invention

An estimated 170 million people worldwide have been infected by hepatitis C virus (HCV). In the next few years, the number of U.S. deaths for HCV-caused liver disease and cancer may overtake deaths caused by Acquired Immune Deficiency Syndrome (AIDS).

The transmission of HCV seems to require blood-to-blood contact. Carrying a single strand of ribonucleic acid (RNA), HCV contains just one gene, coding for a polyprotein that is subsequently cleaved into at least 10 functional proteins. Clearly, the ability to test the blood supply for HCV is of great importance. A sensitive assay that can detect infection at an early stage. Detection of both HCV antigens and antibodies in a single assay would therefore be advantageous.

HCV core detection assays capture HCV antigen from the HCV infected specimens on a solid phase coated with anti-core monoclonal antibodies. The captured core protein is detected by a binding reaction with an anti-core monoclonal antibody labeled with enzyme using standard immunoassay technology. Availability of good monoclonal antibodies to multiple epitopes of HCV core antigen will increase the sensitivity of HCV antigen detection assays. For one thing, multiple antibodies increase the efficiency of the capture of the HCV antigen from specimens onto the solid phase. Second, using multiple antibodies for detection increases the sensitivity of the system by providing a cumulative higher signal. Another advantage is that the use of multiple epitope recognizing antibodies in assays enables the assays to detect specimens infected with different genotypes of the HCV virus.

FR 2 775 690 describes monoclonal antibodies specific for hepatitis C virus (HCV) core protein that binds to an epitope comprising one of the following amino acids of the core protein; 18-25, 29-36 or 57-64.

WO 00/63444 describes a method of directly detecting Hepatitis C virus in a fractionated or non-fractionated serum of a patient, which comprises detecting the virus with primers corresponding to viral RNA encoding core protein.

EP 0 388 232 describes the identification of a new virus, Hepatitis C virus (HCV), which has proven to be the major etiologic agent of blood-borne NANBH. The invention includes the application of the sequences and polypeptides in immunoassays and anti-HCV antibody production.

### Summary of the Invention

The present application describes a library of monoclonal antibodies directed against various regions of the HCV core protein. These antibodies recognize ten (10) different epitopes in the HCV core region and are spread throughout the core protein. The epitopes recognized by these monoclonal antibodies are five to eight (5-8) amino acids ("aa") long. These antibodies in individual pairs or multiple pairs are useful in detecting HCV core protein in patients infected with HCV. Specifically, these antibodies are advantageous in detecting HCV in blood from infected humans. Finally, these antibodies along with properly tailored recombinant proteins can be used for the simultaneous detection of HCV core protein and ant-HCV antibodies.

We have developed a number of anti-HCV core monoclonal antibodies. These antibodies have unique properties in that they recognize short sequences, in the region of HCV core that does not have major epitopes recognized by human anti-HCV antibodies. These antibodies can be useful reagents in detecting HCV core antigen in blood specimens from individuals infected with HCV. The recognition of short epitopes by these monoclonal antibodies and the location of these sequences in the core region of HCV make these monoclonal antibodies very useful in the development of a HCV antigen/antibody combination assay. For the combination assay these monoclonal antibodies are used in combination with core antigen that has been modified to eliminate the ability of these antigens to bind to these monoclonal antibodies but maintain their ability to bind human anti-HCV antibodies. Therefore, the HCV core proteins used to capture the antibodies present in infected serum will not react with the HCV core monoclonal antibodies used in the test to simultaneously capture the antigens present in the infected serum. A combination assay can detect HCV infection earlier than the currently employed HCV antibody assay.

The present invention is directed to a hybridoma cell line corresponding to the ATCC number PTA-3779, antibodies secreted by this hybridoma, the use of those antibodies to detect HCV core protein and to kits containing those antibodies.

### Detailed Description of the Invention

The effectiveness and advantages of the invention are further illustrated by the following examples.

### Example 1

### Immunogens

Immunogens used for raising the antibodies were a full length core recombinant protein ("FLC")(amino acids (aa) 1-191); c22-3, HCV core protein expressed in yeast as an SOD fusion protein (aa 1-120); and large synthetic peptides, such as ODS243(aa 70-110); and keyhole limpet hemacynin (KLH) conjugated 15mer peptides containing sequences from HCV core antigen.

### Example 2

### Immunizing Protocol

The antibodies were generated using standard procedures. See for example, Ed Harlow and David Lane, Antibodies, A Laboratory Manual, 1988, Cold Spring Harbor, Chapter 6. Mice were immunized with the immunogens described herein. The mice were bled and screened with a number of recombinant proteins and peptides from the HCV core sequence. Pure monoclonal antibodies were prepared by protein A affinity chromatography. The fine specificity of the monoclonal antibodies was determined by binding overlapping octapeptides contained in each of the corresponding 15mer peptide. Most antibodies were mapped to a 6-8 amino acid region in the HCV core protein sequence.

### Example 3

### Characterization of the Antibodies

The monoclonal antibodies were characterized by ELISA using overlapping synthetic peptides approximately 15 aa long. The antibodies were further characterized for their fine specificity by peptide ELISA using overlapping octapeptides included in the reactive 15mer peptide. A summary of antibodies generated is included in the following table. The invention is directed to the antibody secreted from the hybridoma with ATCC accession number PTA-3799. The remaining 14 antibodies are included for reference only.

### Table 1

The table below identifies 15 novel antibodies. The invention is directed to the antibody secreted from the hybridoma with ATCC accession number PTA-3799. The remaining 14 antibodies are included for reference only. The antibodies were screened at every stage of antibody development with microtitrewell plates coated with immunogen. The immunogen used to immunize the mice that produced each strain of monoclonal antibody is identified as one of the following: peptide ODS 243, a large peptide further defined in Example 1; "FLC" means full length core antigen, defined in Example 1; or KLH conjugated core peptide #8, a short peptide, further defined herein. The specificity of each to a numbered peptide is shown and the amino acid sequences of each numbered peptide is identified herein. Furthermore, the epitope to which the antibody specifically binds is included in the last column, defined by the amino acids that encode for the epitope.

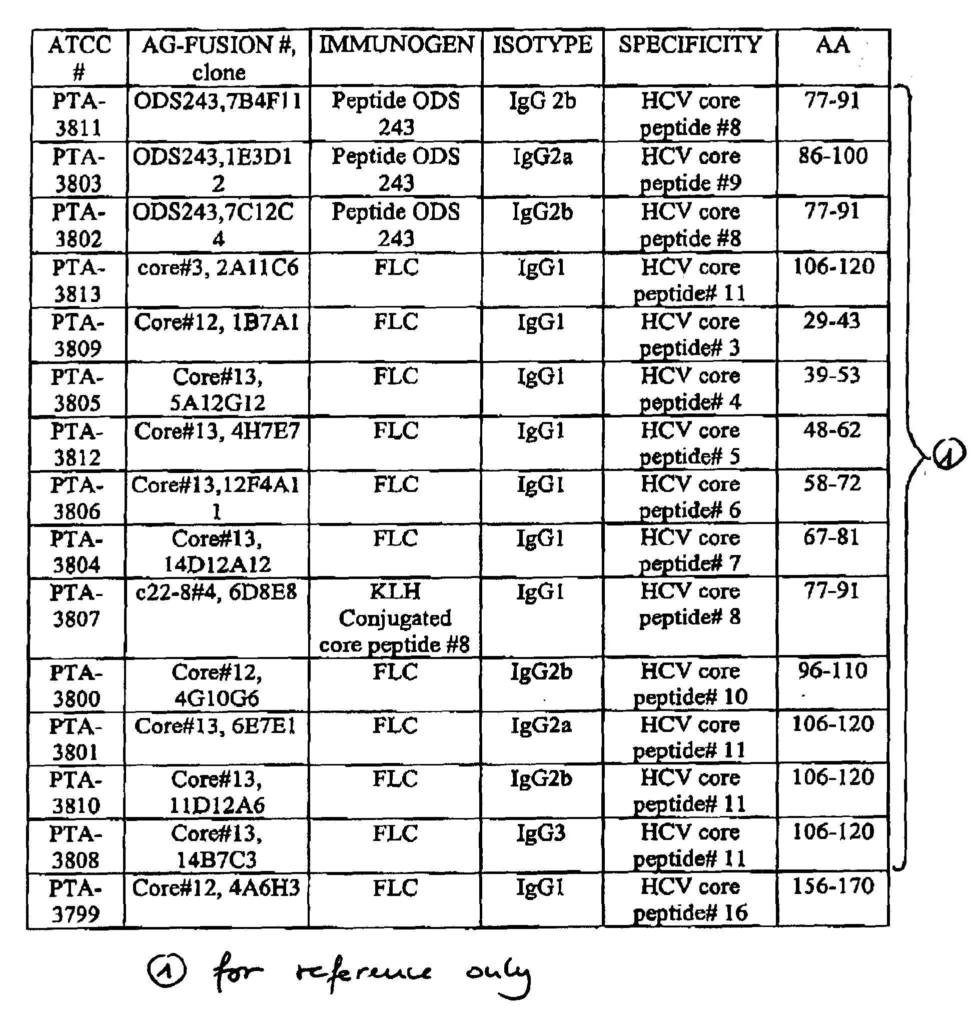

### Reference Table 1

The table below shows HCV core synthetic peptides.

| Peptide ID# | Amino Acid Sequence | HCV protein AA Location | |
|---|---|---|---|
| 0 | MSTNPKPQKKNKRNT | 1-15 | SEQ ID NO.: 1 |
| 1 | KNKRNTNRRPQDVKF | 10-24 | SEQ ID NO.: 2 |
| 2 | QDVKFPGGGQIVGGV | 20-34 | SEQ ID NO.: 3 |
| 3 | QIVGGVYLLPRRCPR | 29-43 | SEQ ID NO.: 4 |
| 4 | RRGPRLGVRATRKTS | 39-53 | SEQ ID NO.: 5 |
| 5 | ATRKTSERSQPRGRR | 48-62 | SEQ ID NO.: 6 |
| 6 | PRGRRQPIPKARRPE | 58-72 | SEQ ID NO.: 7 |
| 7 | KARRPEGRTWAQPGY | 67-81 | SEQ ID NO.: 8 |
| 8 | AQPGYPWPLYGNEGC | 77-91 | SEQ ID NO.: 9 |
| 9 | YGNEGCGWAGWLLSP | 86-100 | SEQ ID NO.: 10 |
| 10 | WLLSPRGSRPSWGPT | 96-110 | SEQ ID NO.: 11 |
| 11 | SWGPTDPRRRSRLNG | 106-120 | SEQ ID NO.: 12 |
| 12 | SRLNGKVIDTLTCGF | 116-130 | SEQ ID NO.: 13 |
| 13 | LTCGFADLMGYIPLV | 126-140 | SEQ ID NO.: 14 |
| 14 | YIPLVGAPLGGAARA | 136-150 | SEQ ID NO.: 15 |
| 15 | GAARALAHGVRVLED | 146-160 | SEQ ID NO.: 16 |
| 16 | RVLEDGVNYATGNLP | 156-170 | SEQ ID NO.: 17 |
| 17 | TGNLPOCSFSIFLLA | 166-180 | SEQ ID NO.: 18 |
| 18 | IFLLALLSCLTVPAS | 176-190 | SEQ ID NO.: 19 |

### Reference Table 2

The table below shows the screening of the antibody identified as PTA-3811 with ELISA plates coated with HCV core peptides.

| Peptide ID # | Optical Density in ELISA |
|---|---|
| 0 | 0 |
| 1 | 0.04 |
| 2 | 0 |
| 3 | 0.01 |
| 4 | 0.01 |
| 5 | 0 |
| 6 | 0 |
| 7 | 0.01 |
| 8 | 2.5 |
| 9 | 0 |
| 10 | 0.01 |
| 11 | 0.01 |
| 12 | 0 |
| 13 | 0.01 |
| 14 | 0,01 |
| 15 | 0 |
| 16 | 0.01 |
| 17 | 0.01 |
| 18 | 0.01 |

### Reference Table 3

Epitope mapping of monoclonal antibody PTA-3811 using overlapping octapeptides.

| Sequence of octapeptides | AA Location | ALU |
|---|---|---|
| (7.9) Biotin -Ahx-TWAQPGYP | 75-82 | 0.57 |
| (7. 10) Biotin-Ahx-WAQPGYPW | 76-83 | 1.05 |
| (8.1) Biotin-Ahx-AQPGYPWP | 77-84 | 0.61 |
| (8.2) Biotin-Ahx-QPGYPWPL | 78-85 | 10.84 |
| (8.3) Biotin-Ahx-PGYPWPLY | 79-86 | 45.89 |
| (8.4) Biotin-Ahx-GYPWPLYG | 80-87 | 43.03 |
| (8.5) Biotin-Ahx-YPWPLYGN | 81-88 | 33.81 |
| (8,6) Biotin-Ahx-PWPLYGNE | 82-89 | 3.11 |
| (8,7) Biotin-Ahx-WPLYGNEG | 83-90 | 0.27 |
| (8.8) Biotin-Ahx-PLYGNEGC | 84-91 | 0.3 |
| (8.9) Biotin-Ahx-LYGNEGCG | 85-92 | 0.42 |
| (9.1) Biotin-Ahx-YGNEGCGW | 86-93 | 0.49 |
| (9.2) Biotin-Ahx-GNEGCGWA | 87-94 | 0.6 |

Monoclonal antibody 7B4F11 reacted with Octapeptides 8.2, 8.3,8.4, 8.5 in a chemiluminiscence ELISA measured in arbitrary light units (ALU). Based on the reactivity of the octapeptides the monoclonal epitope is centered around the sequence PWPL.

## Claims

1. A hybridoma cell line corresponding to the ATCC number PTA-3779.

2. A monoclonal antibody secreted by the hybridoma cell line claimed in claim 1.

3. The use of the monoclonal antibody claimed in claim 2 to detect HCV core antigen.

4. Assay kits that contain the monoclonal antibody claimed in claim 2.

## Patentansprüche

1. Hybridomzelllinie gemäß ATCC-Nummer PTA-3779.

2. Monoklonaler Antikörper, der durch die in Anspruch 1 beanspruchte Hybridomzelllinie sekretiert wird.

3. Verwendung des monoklonalen Antikörpers, der in Anspruch 2 beansprucht ist, um HCV-Kernantigen nachzuweisen.

4. Assaykits, die den monoklonalen Antikörper enthalten, der in Anspruch 2 beansprucht ist.

## Revendications

1. Lignée cellulaire d'hybridome correspondant au numéro ATCC PTA-3779.

2. Anticorps monoclonal sécrété par la lignée cellulaire d' l'hybridome selon la revendication 1.

3. Utilisation de l'anticorps monoclonal selon la revendication 2 pour détecter un antigène du noyau du VHC.

4. Kits de test qui contiennent l'anticorps monoclonal selon la revendication 2.


### SEQUENCE LISTING

<110> Ortho-Clinical Diagnostics, Inc.
<120> HCV Anti-Core Monoclonal Antibodies
<130> CDS0286
<150> US 60/337453
   <151> 2001-11-05
<160> 19
<170> PatentIn version 3.1
<210> 1
   <211> 15
   <212> PRT
   <213> Hepatitis C virus
<400> 1
   Met Ser Thr Asn Pro Lys Pro Gln Lys Lys Asn Lys Arg Asn Thr
   1 5 10 15
<210> 2
   <211> 15
   <212> PRT
   <213> Hepatitis C virus
<400> 2
   Lys Asn Lys Arg Asn Thr Asn Arg Arg Pro Gln Asp Val Lys Phe
   1 5 10 15
<210> 3
   <211> 15
   <212> PRT
   <213> Hepatitis C virus
<400> 3
   Gln Asp Val Lys Phe Pro Gly Gly Gly Gln Ile Val Gly Gly Val
   1 5 10 15
<210> 4
   <211> 15
   <212> PRT
   <213> Hepatitis C virus
<400> 4
   Gln Ile Val Gly Gly Val Tyr Leu Leu Pro Arg Arg Gly Pro Arg
   1 5 10 15
<210> 5
   <211> 15
   <212> PRT
   <213> Hepatitis C virus
<400> 5
   Arg Arg Gly Pro Arg Leu Gly Val Arg Ala Thr Arg Lys Thr Ser
   1 5 10 15
<210> 6
   <211> 15
   <212> PRT
   <213> Hepatitis C virus
<400> 6
   Ala Thr Arg Lys Thr Ser Glu Arg Ser Gln Pro Arg Gly Arg Arg
   1 5 10 15
<210> 7
   <211> 15
   <212> PRT
   <213> Hepatitis C virus
<400> 7
   Pro Arg Gly Arg Arg Gln Pro Ile Pro Lys Ala Arg Arg Pro Glu
   1 5 10 15
<210> 8
   <211> 15
   <212> PRT
   <213> Hepatitis C virus
<400> 8
   Lys Ala Arg Arg Pro Glu Gly Arg Thr Trp Ala Gln Pro Gly Tyr
   1 5 10 15
<210> 9
   <211> 15
   <212> PRT
   <213> Hepatitis C virus
<400> 9
   Ala Gln Pro Gly Tyr Pro Trp Pro Leu Tyr Gly Asn Glu Gly Cys
   1 5 10 15
<210> 10
   <211> 15
   <212> PRT
   <213> Hepatitis C virus
<400> 10
   Tyr Gly Asn Glu Gly Cys Gly Trp Ala Gly Trp Leu Leu Ser Pro
   1 5 10 15
<210> 11
   <211> 15
   <212> PRT
   <213> Hepatitis C virus
<400> 11
   Trp Leu Leu Ser Pro Arg Gly Ser Arg Pro Ser Trp Gly Pro Thr
   1 5 10 15
<210> 12
   <211> 15
   <212> PRT
   <213> Hepatitis C virus
<400> 12
   Ser Trp Gly Pro Thr Asp Pro Arg Arg Arg Ser Arg Leu Asn Gly
   1 5 10 15
<210> 13
   <211> 15
   <212> PRT
   <213> Hepatitis C virus
<400> 13
   Ser Arg Leu Asn Gly Lys Val Ile Asp Thr Leu Thr Cys Gly Phe
   1 5 10 15
<210> 14
   <211> 15
   <212> PRT
   <213> Hepatitis C virus
<400> 14
   Leu Thr Cys Gly Phe Ala Asp Leu Met Gly Tyr Ile Pro Leu Val
   1 5 10 15
<210> 15
   <211> 15
   <212> PRT
   <213> Hepatitis C virus
<400> 15
   Tyr Ile Pro Leu Val Gly Ala Pro Leu Gly Gly Ala Ala Arg Ala
   1 5 10 15
<210> 16
   <211> 15
   <212> PRT
   <213> Hepatitis C virus
<400> 16
   Gly Ala Ala Arg Ala Leu Ala His Gly Val Arg Val Leu Glu Asp
   1 5 10 15
<210> 17
   <211> 15
   <212> PRT
   <213> Hepatitis C virus
<400> 17
   Arg Val Leu Glu Asp Gly Val Asn Tyr Ala Thr Gly Asn Leu Pro
   1 5 10 15
<210> 18
   <211> 15
   <212> PRT
   <213> Hepatitis C virus
<400> 18
   Thr Gly Asn Leu Pro Gly Cys Ser Phe Ser Ile Phe Leu Leu Ala
   1 5 10 15
<210> 19
   <211> 15
   <212> PRT
   <213> Hepatitis C virus
<400> 19
   Ile Phe Leu Leu Ala Leu Leu Ser Cys Leu Thr Val Pro Ala Ser
   1 5 10 15

### SEQUENCE LISTING

<110> Ortho-Clinical Diagnostics, Inc.
<120> HCV Anti-Core Monoclonal Antibodies
<130> P032220EP
<140> 02257622.7
   <141> 2002-11-04
<150> US 60/337453
   <151> 2001-11-05
<160> 19
<170> Patentln version 3.1
<210> 1
   <211> 15
   <212> PRT
   <213> Hepatitis C virus
<400> 1
   Met Ser Thr Asn Pro Lys Pro Gln Lys Lys Asn Lys Arg Asn Thr
   1               5                   10                  15
<210> 2
   <211> 15
   <212> PRT
   <213> Hepatitis C virus
<400> 2
   Lys Asn Lys Arg Asn Thr Asn Arg Arg Pro Gln Asp Val Lys Phe
   1               5                   10                  15
<210> 3
   <211> 15
   <212> PRT
   <213> Hepatitis C virus
<400> 3
   Gln Asp Val Lys Phe Pro Gly Gly Gly Gln Ile Val Gly Gly Val
   1               5                   10                  15
<210> 4
   <211> 15
   <212> PRT
   <213> Hepatitis C virus
<400> 4
   Gln Ile Val Gly Gly Val Tyr Leu Leu Pro Arg Arg Gly Pro Arg
   1               5                   10                  15
<210> 5
   <211> 15
   <212> PRT
   <213> Hepatitis C virus
<400> 5
   Arg Arg Gly Pro Arg Leu Gly Val Arg Ala Thr Arg Lys Thr Ser
   1               5                   10                  15
<210> 6
   <211> 15
   <212> PRT
   <213> Hepatitis C virus
<400> 6
   Ala Thr Arg Lys Thr Ser Glu Arg Ser Gln Pro Arg Gly Arg Arg
   1               5                   10                  15
<210> 7
   <211> 15
   <212> PRT
   <213> Hepatitis C virus
<400> 7
   Pro Arg Gly Arg Arg Gln Pro Ile Pro Lys Ala Arg Arg Pro Glu
   1 5 10 15
<210> 8
   <211> 15
   <212> PRT
   <213> Hepatitis C virus
<400> 8
   Lys Ala Arg Arg Pro Glu Gly Arg Thr Trp Ala Gln Pro Gly Tyr
   1 5 10 15
<210> 9
   <211> 15
   <212> PRT
   <213> Hepatitis C virus
<400> 9
   Ala Gln Pro Gly Tyr Pro Trp Pro Leu Tyr Gly Asn Glu Gly Cys
   1 5 10 15
<210> 10
   <211> 15
   <212> PRT
   <213> Hepatitis C virus
<400> 10
   Tyr Gly Asn Glu Gly Cys Gly Trp Ala Gly Trp Leu Leu Ser Pro
   1 5 10 15
<210> 11
   <211> 15
   <212> PRT
   <213> Hepatitis C virus
<400> 11
   Trp Leu Leu Ser Pro Arg Gly Ser Arg Pro Ser Trp Gly Pro Thr
   1               5                   10                  15
<210> 12
   <211> 15
   <212> PRT
   <213> Hepatitis C virus
<400> 12
   Ser Trp Gly Pro Thr Asp Pro Arg Arg Arg Ser Arg Leu Asn Gly
   1               5                   10                  15
<210> 13
   <211> 15
   <212> PRT
   <213> Hepatitis C virus
<400> 13
   Ser Arg Leu Asn Gly Lys Val Ile Asp Thr Leu Thr Cys Gly Phe
   1               5                   10                  15
<210> 14
   <211> 15
   <212> PRT
   <213> Hepatitis C virus
<400> 14
   Leu Thr Cys Gly Phe Ala Asp Leu Met Gly Tyr Ile Pro Leu Val
   1               5                   10                  15
<210> 15
   <211> 15
   <212> PRT
   <213> Hepatitis C virus
<400> 15
   Tyr Ile Pro Leu Val Gly Ala Pro Leu Gly Gly Ala Ala Arg Ala
   1               5                   10                  15
<210> 16
   <211> 15
   <212> PRT
   <213> Hepatitis C virus
<400> 16
   Gly Ala Ala Arg Ala Leu Ala His Gly Val Arg Val Leu Glu Asp
   1               5                   10                  15
<210> 17
   <211> 15
   <212> PRT
   <213> Hepatitis C virus
<400> 17
   Arg Val Leu Glu Asp Gly Val Asn Tyr Ala Thr Gly Asn Leu Pro
   1               5                   10                  15
<210> 18
   <211> 15
   <212> PRT
   <213> Hepatitis C virus
<400> 18
   Thr Gly Asn Leu Pro Gly Cys Ser Phe Ser Ile Phe Leu Leu Ala
   1               5                   10                  15
<210> 19
   <211> 15
   <212> PRT
   <213> Hepatitis C virus
<400> 19
   Ile Phe Leu Leu Ala Leu Leu Ser Cys Leu Thr Val Pro Ala Ser
   1               5                   10                  15